# EUROPÄISCHE PATENTSCHRIFT

(11) **EP 1 940 848 B1**
(45) Veröffentlichungstag und Bekanntmachung des Hinweises auf die Patenterteilung: **09.12.2009**
(21) Anmeldenummer: 06791409.3
(22) Anmeldetag: 25.09.2006
(51) Int. Cl.: C07D 493/04, A61K 31/422, A61P 35/00

(54) **ANTIMITOTISCHE RHIZOXIN-DERIVATE AUS BURKHOLDERIA RHIZOXINA, VERFAHREN ZU IHRER HERSTELLUNG UND DEREN VERWENDUNG**
ANTIMITOTIC RHIZOXIN DERIVATIVES OF BURKHOLDERIA RHIZOXINA, METHOD FOR PRODUCING SAID DERIVATIVES AND USE THEREOF
DERIVES DE RHIZOXINE ANTIMITOTIQUES DE BURKHOLDERIA RHIZOXINA, PROCEDE DE PRODUCTION DESDITS DERIVES ET LEUR UTILISATION

(30) Priorität: 06.10.2005 DE 102005048556
(43) Veröffentlichungstag der Anmeldung: 09.07.2008
(73) Patentinhaber: Leibniz-Institut für Naturstoff-Forschung und Infektionsbiologie e.V. Hans-Knöll-Institut, 07745 Jena (DE)
(72) Erfinder: SCHERLACH, Kirstin, 04105 Leipzig (DE); PARTIDA-MARTINEZ, Laila, 07743 Jena (DE); HERTWECK, Christian, 04105 Leipzig (DE)
(74) Vertreter: Donath, Dirk
(86) Internationale Anmeldenummer: PCT/DE2006/001708
(87) Internationale Veröffentlichungsnummer: WO 2007/041986

(56) Entgegenhaltungen:
- DE-B3-102005 026 417
- JP-A- 3 101 680
- JP-A- 6 193 186
- KIYOTO, SUMIO ET AL: "A new antitumor complex, WF-1360, WF-1360A, B, C, D. E and F" JOURNAL OF ANTIBIOTICS , 39(6), 762-72 CODEN: JANTAJ; ISSN: 0021-8820, 1986, XP009078574
- ROBERGE, MICHEL ET AL: "Cell-based screen for antimitotic agents and identification of analogues of rhizoxin, eleutherobin, and paclitaxel in natural extracts" CANCER RESEARCH , 60(18), 5052-5058 CODEN: CNREA8; ISSN: 0008-5472, 2000, XP002418830

## Beschreibung

Die Erfindung betrifft vier neue Sekundärmetabolite des Endosymbionten *Burkholderia rhizoxina,* ein Verfahren zur Isolation dieser Verbindungen aus Kulturen des Bakteriums und die Verwendung der Substanzen.

Maligne Tumore sind neben Erkrankungen des Herz-Kreislauf-Systems die zweithäufigste Todesursache in Deutschland (DKFZ, 2000).

Trotz intensiver Forschung in den letzten Jahren stellt die Behandlung einiger Krebsarten immer noch eine große Herausforderung dar.

Rhizoxin ist ein makrocyclisches Polyketid mit antimitotischer Wirkung, welches aus Pilzen der Gattung *Rhizopus* isoliert wurde (Iwasaki, S. et al. J. Antibiot., 1984, 37, 354-362). Es weist eine starke Aktivität gegen eine Reihe humaner Tumorzelllinien, insbesondere auch gegen Vincristin-resistente Zellen, auf und hat daher großes Interesse als potentielles Chemotherapeutikum geweckt. Die Wirkung beruht auf einer Bindung an β-Tubulin eukaryotischer Zellen, was zur Hemmung der Assemblierung der Mikrotubuli führt. Kürzlich konnten wir zeigen, dass Rhizoxin nicht von *Rhizopus* gebildet wird, sondern von bakteriellen Endosymbionten des Pilzes (L. Partida-Martinez und C. Hertweck, Nature, 2005, 437, 884-888). Durch Kultivierung des Endosymbionten konnte die Produktion von Rhizoxin und von Derivaten der Substanz erheblich gesteigert werden.

Aus den Schriften Kiyoto, S. et al., Journal of Antibiotics, 1986, 39(6), 762-772, Roberge, M. et al., Cancer Research, 2000, 60(18), 5052-50-58, JP 06 193186 A und JP 03 101680 A sind Rhizoxin-Derivate bekannt, welche als Anti-Krebsmittel und Fungizide wirksam sind. Diese Derivate weisen folgende Strukturformeln auf: 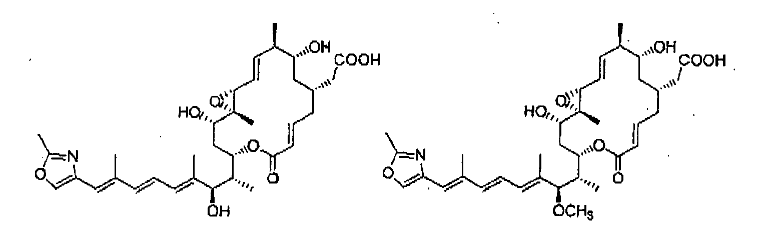 und

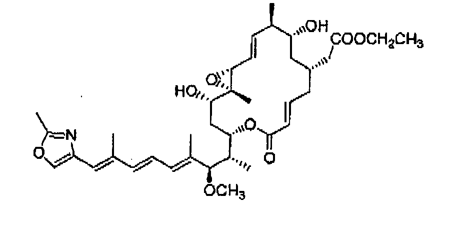

Insbesondere aus Kiyoto, S. et al., Journal of Antibiotics, 1986, 39(6), 762-772 ist bekannt, dass der Ethylester schwächer wirksam gegen P388 Leukämiezellen ist als Rhizoxin, was zeigt, dass Ester bekannter Verbindungen / von Rhizoxin schwächere Aktivitäten aufweisen, als freie Säuren dieser Verbindungen.

DE 10 2005 026 417 B3 offenbart ein Verfahren zur Herstellung von Rhizoxin und Derivaten von Rhizoxin, bei dem bakterielle Symbionten aus Rhizopus sp. in Form von *Burkholderia Rhizoxina* DSM 17360 oder andere *Burkholderia Rhizoxina-Stämme* isoliert, kultiviert und zur fermentativen Herstellung von Rhizoxin und/oder Derivaten des Rhizoxins in Submerskultur mit anschließender Isolation des Rhizoxins und/oder seiner Derivate aus dem Kulturüberstand verwendet werden.

Aufgabe der Erfindung ist es, neue, antimitotisch wirksame Rhizoxin-Derivate, welche stärker wirksam als die aus dem Stand der Technik bekannten seco-Verbindungen sind, sowie ein Verfahren zu ihrer Herstellung zur Verfügung zu stellen. Desweiteren sollen deren Verwendungen angegeben werden.

Diese Aufgabe wird erfindungsgemäß durch Substanzen gemäß Anspruch 1, ein Verfahren gemäß Anspruch 2 und Verwendungen gemäß den Ansprüche 6 bis 8 gelöst. Vorteilhafte Ausgestaltungen werden in den nachgeordneten Ansprüchen angegeben.

Die Herstellung der Substanzen gemäß der Formeln **2-4** erfolgt durch Kultivierung des endosymbiontischen Bakterienstammes *Burkholderia rhizoxina,* anschließender Extraktion der Kultur und Isolation der Verbindungen mittels chromatographischer Verfahren.

Dabei wird *Burkholderia rhizoxina* DSM 17360 auf einem Flüssigmedium als Schüttelkultur kultiviert und der Kulturansatz anschließend mit organischen Lösungsmitteln extrahiert.

Danach wird der Extrakt mittels Größenausschlußchromatographie an Dextrangelen (Sephadex LH-20) fraktioniert. Eine Endreinigung der Substanzen erfolgt mittels präparative HPLC unter Verwendung einer RP-18 Phase und Acetonitril / Wasser-Gemischen im Gradientenmodus.

Die Struktur der Verbindungen **2-4** wird durch IR-Spektroskopie, hochaufgelöste Massenspektrometrie und 1D und 2D NMR-Spektroskopie aufgeklärt.

Die erfindungsgemäßen Substanzen **2-4** weisen sehr starke antiproliferative und zytotoxische Wirkung (bzw. bei L-929 Mausfibroblasten, K-562 Humanleukämiezellen und HeLa Human-Cervix-Carcinomlinie) sowie antifungale Aktivität (bspw. gegen *Glomerella cingulata, Penicillium notatum, Fusarium culmorum, Hamigera avellanea, Aspergillus fumigatus*) auf. (Tabelle 1)

Die Substanzen Substanzen **2-4** eignen sich auf Grund ihrer starken antiproliferativen und zytotoxischen Eigenschaften sehr gut zur Anwendung als Chemotherapeutika zur Behandlung von Krebserkrankungen.

Die gute antifungale Wirkung der Substanzen **2-4** ermöglicht ferner die Verwendung zur Therapie von Pilzinfektionen.

Die Verbindungen **(2 - 4)** können als solche in Substanz oder als pharmazeutische Zubereitungen in Verbindung mit üblichen Hilfsstoffen verwendet werden.

### Ausführungsbeispiele

Die Kultivierung von *Burkholderia rhizoxina* DSM 17360 erfolgt durch Fermentation auf einem Flüssigmedium (Zusammensetzung: MaisStärke 1%, Glycerin 0.5%, Hefeextrakt 1%, Maisquellwasser 1%, CaCO₃ 1%) als Schüttelkultur bei 30 °C (4 d). Der gesamte Kulturansatz wird mit Ethylacetat unter Rühren extrahiert und anschließend filtriert. Die Prozedur wird zweimal wiederholt. Die vereinigten Extrakte werden unter Verwendung von Natriumsulfat getrocknet und eingeengt. Der erhaltene Extrakt wird in Methanol gelöst und mittels Größenausschlußchromatographie an Sephadex LH-20 fraktioniert (Eluent Methanol). Die Reindarstellung der Substanzen 1 bis 4 erfolgt durch präparative HPLC unter Verwendung einer RP-18 Phase und Acetonitril / Wasser-Gemischen (Methode: MeCN / H₂O 25:75 5 min, dann in 35 min auf MeCN / H₂O 80:20, dann in 5 min auf MeCN 100%, Detektion bei 31 nm).

### Substanz 2:

Weißes Pulper. IR (ATR, Festsubstanz) vₘₐₓ/cm⁻¹ 2977, 2935, 2924, 1705,1652,1577,1437,1377,1260,1202,1152,1105, 1048,1007,966, 863, 827, 780, 748, 702. ¹H NMR (300 MHz) und ¹³C NMR (75 MHz) in d-Chloroform [siehe Tabelle 2]. (+)-ESI-MS *mlz* 628 [M+H]⁺, *mlz* 650 [M+Na]⁺. HRESI-MS: *m*/*z* [M+H]⁺ = 628,3478 (berechnet für C₃₅H₅₀NO₉ 628.3486)

### Substanz 3:

Weißes Pulver. IR (ATR, Festsubstanz) νₘₐₓ/cm⁻¹ 2960, 2938, 2928, 1710, 1654, 1577, 1437, 1367, 1275, 1199, 1151, 1108, 1084, 1048, 1008, 971, 862, 827, 753, 706. ¹H NMR (300 MHZ) und ¹³C NMR (75 MHz) in d-Chloroform [siehe Tabelle 2]. (+)-ESI-MS *m*/*z* 642 [M-H]⁺, *mlz* 664 [M+Na]⁺. HRESI-MS: *m*/*z* [M+H]⁺ = 642.3612 (berechnet für C₃₆H₅₂NO₉ 642.3637)

### Substanz 4:

Weißes Pulver. IR (ATR, Festsubstanz) νₘₐₓ/cm^{-1 1}H NMR (300 MHz) und ¹³C NMR (75 MHz) in d-Methanol [siehe Tabelle 2]. (+)-ESI-MS *mlz* 642 [M+H]⁺, *mlz* 664 [M+Na]⁺. HRESI-MS: *mlz* [M+Na]⁺ = 664.3434 (berechnet für C₃₆H₅₁NO₉Na 664.3456)

**Tabelle 1**

| Substanz | L-929 GI₅₀ [µg/ml] | K-562 GI₅₀ [µg/ml] | HeLa CC₅₀ [µg/ml] |
|---|---|---|---|
| 1 | | 1.5 x 10⁻² | 9x10⁻² |
| 2 | 5x10⁻² | 3x10⁻⁵ | 2,8x10⁻⁴ |
| 3 | 5x10⁻² | <3x10⁻⁵ | <3x10⁻⁵ |
| 4 | 1,2x10⁻² | <3x10⁻⁵ | 2x10⁻³ |

Die Untersuchung der antiproliferativen und zytotoxischen Wirksamkeit der Substanzen 1-4 wurde entsprechend der in der Literatur beschriebenen Methode durchgeführt (H.M. Dahse, B. Schlegel, U. Gräfe, Pharmazie 2001, 56, 489-491). Die Bestimmung der antifungalen Aktivität erfolgte mittels Agardiffusionstest.

**Tabelle 2**

| position | 1 | 1 | 2 | 2 | 3 | 3 | 4 | 4 |
|---|---|---|---|---|---|---|---|---|
| | δ_{H} (J [Hz]) | δ_{C} | δ_{H} (J [Hz]) | δ_{C} | δ_{H}(J [Hz]) | δ_{C} | δ_{H} (J [Hz]) | δ_{C} |
| 1 | - | 167.0 | - | 165.7 | - | 165.1 | - | 166,9 |
| 2 | 5.78 d (15.6) | 126.2 | 5.70 d (15.6) | 124.6 | 5.670(15.6) | 124.9 | 5.77 d (15.6) | 126.2 |
| 3 | 6.79 ddd (15.6, 8.1, 7,5) | 147.7 | 6,78 ddd (15.5, 9.0. 6.5) | 147.1 | 6.74 ddd (15.5, 9.0, 6.6) | 146.4 | 6.78 ddd (15.6, 8.2, 7.8) | 147.6 |
| 4 | 2.50m* | 36.8 | 2.40 m | 37,7 | 2.39 m * | 37.5 | 2.47 m | 38.8 |
| | 2.15 m | | 2.06 m* | | 2.06 m | | 2.13m | |
| 5 | 2.2a m | 32.8 | 2.21 m | 32.2 | 2.21 m | 32.1 | 2.28 m | 33.0 |
| 5a | 2.50 dd * | 41.3 | 2.52 dd (15.8. 6.9) | 40.5 | 2.51 dd (15.8, 6.8) | 40.4 | 2.52 dd (15.2, 5.6) | 41.2 |
| | 2.35 dd | | 2.35 d (6.9) | | 2.34 dd (15.7, 6.9)* | | 2.36 dd (15.2, 8.2) | |
| 5b | - | 176.6 | - | 173.5 | - | 173.5 | - | 174.8 |
| 6 | 1.75 m | 39.2 | 1.75 dd (14.5, 5.9) | 37.9 | 1.71 m | 37.8 | 1.71 m | 39.1 |
| | 1.10 m | | 1.09m | | 1,09 m | | 1.05m | |
| 7 | 3,13 m | 73.9 | 3.20 m | 74.3 | 3.16 m | 74.2 | 3.11m | 73.9 |
| 8 | 2.02m | 46.8 | 2.03 m | 45.5 | 2,02 m | 45.5 | 2.00 m* | 46.8 |
| 8a | 1.02 d (6.0)* | 17.7 | 1.03 d (6.65) | 17.0 | 1,02 d (6.6) | 17.0 | 1.02 d (6.7) | 17.7 |
| 9 | 5.45 dd (15.6; 9.2) | 142.3 | 5.51 dd (15.6; 9.4) | 141.6 | 6,48 dd (15.6; 9.4) | 141.4 | 5.43 dd (15.6;9.3) | 142.3 |
| 10 | 5.16 dd (15.7; 8,1) | 127.3 | 5.14 dd (15.6: 8.4) | 125.4 | 5.12 dd (15.6; 8.5) | 125.6 | 15.18 dd (15.6; 8.2) | 127.3 |
| 11 | 3.00 (8.2) | 63.4 | 3.16 d (8.5) | 64.2 | 3.11 d (8.3) | 63.9 | 3.00 d (8.3) | 83.3 |
| 12 | | 66.3 | | 65.6 | | 65.6 | | 68.2 |
| 12a | 7.29 s | 11.2 | 1.33 s | 11.1 | 1.31 s | 11.1 | 1.29 s | 11.2 |
| 13 | 2.96 dd (11.0; 2.7) | 79.5 | 3.09 dd (10.2, 3.6) | 78.2 | 3.00 dd (10.8, 2.8) | 78.3 | 2.94 dd (11.0; 2.6) | 79.5 |
| 14 | 2.03 m * | 34.0 | 1.95 m | 33.1 | 1.94 m | 31.8 | 1.95 m* | 34.0 |
| | 1.80m | | 1.89m | | 1.78 m | | 1.76 m | |
| 15 | 4,76 m | 75.3 | 4.85m | 74.1 | 4.76 dd (9.9, 3.6) | 73.3 | 4.75 dd (9.7; 3.5) | 74.9 |
| 16 | 2,05 m | 41.4 | 1.98 m * | 40.3 | 2.09m | 39.4 | 2.08 m | 40.6 |
| 16a | 0.99 d (6.5) * | 10.3 | 0.94 d (6.8) | 9.6 | 0.97d(6.8) | 10.2 | 1.00 d (6.8) | 10.6 |
| 17 | 3.80 d (8.8) | 60.7 | 3.88 d (6.0) | 77.2 | 3.21d(8.6) | 89.2 | 3.33 d (8.9) | 90.8 |
| 17-OCH3 | - | - | - | - | 3.13s | 56.2 | 3.17s | 56.5 |
| 18 | - | 140.7 | - | 138.2 | - | 136.3 | - | 138. |
| 18a | 1.89 s | 12.0 | 1.83 s | 12.9 | 1.82 s | 11.6 | 1.64 s | 11.7 |
| 19 | 6.14d (10.9) | 128.4 | 8.17 d (10.8) | 126.6 | 6.06 d (10.8) | 129.2 | 6.22 d (10.9) | 131.3 |
| 20 | 6.65dd(15.1; 10.8) | 125.9 | 6.54dd (15.1, 10.7) | 124.3 | 6.57 dd (16.1, 10.7) | 124.1 | 8.71 dd (15.6; 11.0) | 127.4 |
| 21 | 6.40 d (15.2) | 130.3 | 6.35 d (15,2) | 137,0 | 6.34 d (15.2) | 137.6 | 7.27 d (15.3) | 132.8 |
| 22 | - | 139.0 | - | 136.9 | - | 136.9 | - | 137,2 |
| 22a | 209 s | 14.7 | 2.11 s | 14.4 | 2.12 s | 14.3 | 2.04 s | 21.0 |
| 23 | 6.21 s | 120.9 | 8.22 s | 120.5 | 6.23s | 120.7 | 5.78 s | 118.8 |
| 24 | - | 139.5 | - | 138.8 | - | 138.7 | - | 139.1 |
| 25 | 7.79 s | 137.7 | 7.50 s | 135.9 | 7.50 s | 135.9 | 7.74 s | 138. |
| 26 | - | 182,9 | - | 160.9 | - | 160.9 | - | 163.2 |
| 26a | 2.43 s | 13.4 | 2.43 s | 13.8 | 2.43 s | 13.8 | 2.43 | 13.5 |
| 27 | - | - | 3.68s | 51.7 | 3.67 s | 51.7 | 3.87 s | 52.0 |

| | | | | | | | | |
|---|---|---|---|---|---|---|---|---|
| *Partial overlapping of signals | | | | | | | | |

## Patentansprüche

1. Substanzen der Formeln 2-4

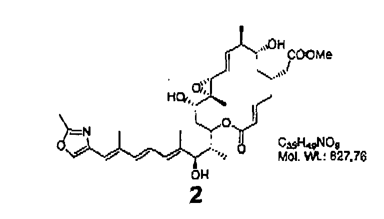

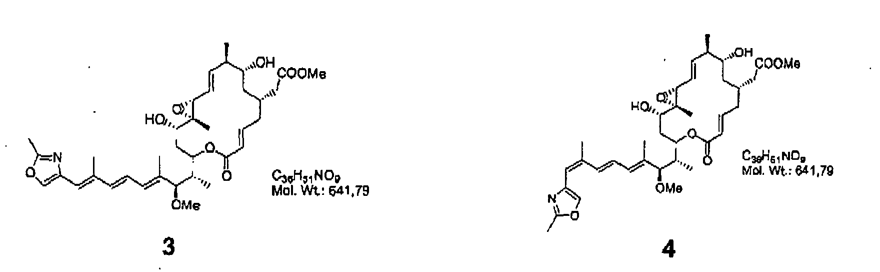

2. Verfahren zur Herstellung der Substanzen **(2-4)** gemäß Anspruch 1, **dadurch gekennzeichnet, dass** ein Endosymbiont auf einem flüssigen Nährmedium kultiviert wird und die Substanzen aus der Kulturbrühe isoliert werden.

3. Verfahren gemäß Anspruch 2, **dadurch** gekenntzeichnet, dass der Endosymbiont *Burkholderia rhizoxina* ist.

4. Verfahren gemäß Anspruch 3, **dadurch gekennzeichnet, dass** der Endosymbiont *Burkholderia rhizoxina* DSM 17360 ist.

5. Pharmazeutische Formulierungen, welche die Substanzen **2-4** enthalten.

6. Verwendung der Verbindungen **2-4** für die Herstellung von zytostatisch wirksamen Therapeutika.

7. Verwendung der Verbindungen **2-4** für die Herstellung von zytotoxisch wirksamen Therapeutika.

8. Verwendung der Verbindungen **2-4** für die Herstellung von antifungal wirksamen Therapeutika.

## Claims

1. Substances of the formulas 2-4

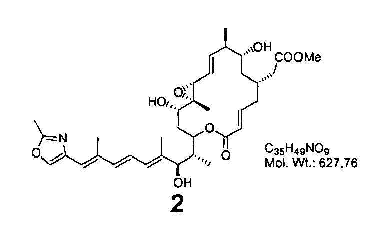

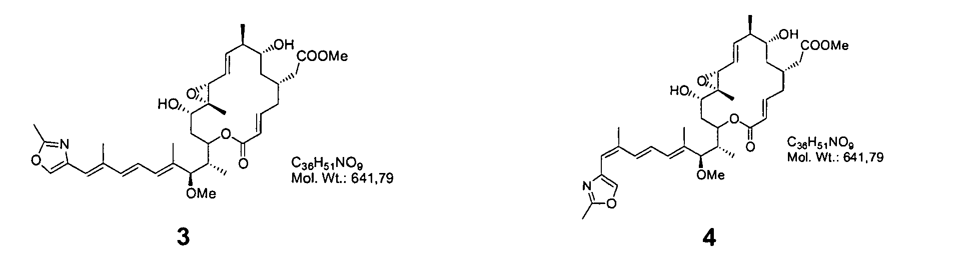

2. Method for producing the substances **(2-4)** according to claim 1, wherein an endosymbiont is cultivated on a liquid culture medium and said substances are isolated from the culture solution.

3. Method according to claim 2, wherein the endosymbiont is *Burkholderia rhizoxina.*

4. Method according to claim 3, wherein the endosymbiont is *Burkholderia rhizoxina* DSM 17360.

5. Pharmaceutical formulations that contain the substances **2-4.**

6. Use of the compounds **2-4** for the production of therapeutics that have a cytostatic effect.

7. Use of the compounds **2-4** for the production of therapeutics that have a cytotoxic effect.

8. Use of the compounds **2-4** for the production of therapeutics that have an antifungal effect.

## Revendications

1. Substances des formules 2-4

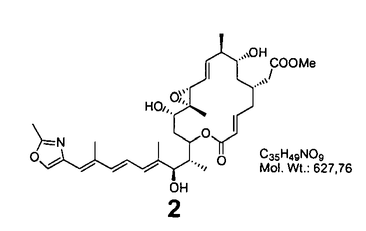

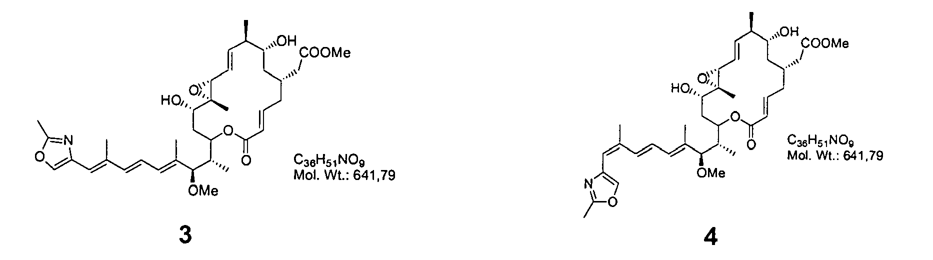

2. Le procédé pour la production des substances **(2-4)** suivant la revendication 1 est **caractérisé en ce qu'**un endosymbionte est cultivé sur un milieu nutritif **liquide** et les substances sont isolées du bouillon de culture.

3. Le procédé suivant la revendication 2 est **caractérisé en ce que** le endosymbionte est *Burkholderia rhizoxina.*

4. Le procédé suivant la revendication 3 est **caractérisé en ce que** le endosymbionte est *Burkholderia rhizoxina* DSM 17360.

5. Formulations pharmaceutiques contenant les substances **2-4.**

6. Utilisation des composés **2-4** pour la production des agents thérapeutiques d'efficacité cytostatique.

7. Utilisation des composés **2-4** pour la production des agents thérapeutiques d'efficacité cytotoxique.

8. Utilisation des composés **2-4** pour la production des agents thérapeutiques d'efficacité antifongique.
